# EUROPÄISCHE PATENTANMELDUNG

(11) **EP 4 461 386 A1**
(43) Veröffentlichungstag der Anmeldung: **13.11.2024**
(21) Anmeldenummer: 24174899.5
(22) Anmeldetag: 08.05.2024
(51) Int. Cl.: A63B 69/00, A61B 17/16, A61C 3/02, A63B 43/00, A63B 63/00, A63B 71/02, A63B 71/06, A63B 102/24, E04H 12/22

(54) **EISMAUS**

(30) Priorität: 08.05.2023 BE 202300029
(71) Anmelder: CJ Beteiligungsgesellschaft UG (haftungsbeschränkt), 01159 Dresden (DE)
(72) Erfinder: Jehle, Paul, 88214 Ravensburg (DE); Jehle, Claudius, 80469 München (DE)
(74) Vertreter: Rössler, Thomas

(57) **Zusammenfassung**

Die vorliegende Erfindung bezieht sich auf Vorrichtungen und Verfahren zum präzisen Lokalisierungen und Bestimmen der Ausrichtung von unter Oberflächen verborgenen Objekten mittels Magneten, so beispielsweise Unterputzdosen im Gebäudebau und der - instandhaltung, als auch im Eissport (Eishockey, Eiskunstlauf) unter Eisflächen, im letzteren Anwendungsfall beispielsweise um Aufnahmehülsen für Eissporttore präzise in Ort und Ausrichtung zu bestimmen, um diese dann bereits im ersten Versuch, und ohne Beschädigung der Hülse, beispielsweise durch einen Eisbohrer, erfolgreich freilegen zu können.

## Beschreibung

### Gebiet der Erfindung

Die vorliegende Erfindung bezieht sich auf das Gebiet der Bautechnik, der Eis- und Wintersporttechnik und der Medizintechnik. Weitere Anwendungsfelder der Erfindung sind denkbar.

### Technischer Hintergrund

Eisflächen für sportliche Tätigkeiten erfreuen seit jeher die Menschen verschiedenster Kulturen. Sowohl natürlich entstandene als auch künstliche und künstlich instandgehaltene Eisflächen werden dabei von Eissportlern mit großer Begeisterung genutzt. Beispielhaft seien Eiskunstlauf und Eishockey genannt.

Im praktischen Betrieb stellt sich für die Betreiber von Eissportanlagen und insbesondere deren Eismeister, aber auch bereits für Hobbysportler ein großes Problem:
Verschiedene Eissportarten sind auf Objekte angewiesen, welche ortsfest in das Eis eingebracht werden müssen. In einem prägnanten Beispiel müssen Torpfosten, beispielsweise für Eishockey, am Untergrund ausgerichtet und hierzu in das Eis eingebracht werden. Dazu werden im Stand der Technik häufig Hülsen eingesetzt, welche im Eis bzw. Untergrund verbleiben, auch wenn beispielsweise Pfosten zwecks Erneuerung des Eis entfernt werden.

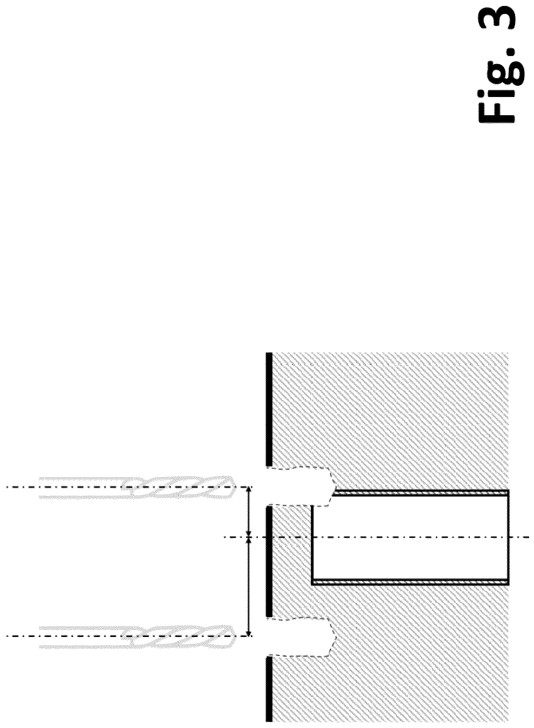

Nachdem die Eisfläche erneuert wurde, ist die neue Eisfläche jedoch verschlossen, samt dem Zugang zur Hülse. Regelmäßig setzen Eismeister im Stand der Technik hierzu spezielle Eisbohrmaschinen ein, um die Hülsen wieder aufzubohren und so zur erneuten Aufnahme eines Pfostens freizulegen. Häufig sind diese Bohrer nur geringfügig kleiner im Durchmesser als die Hülse, was sinnvoll ist, damit der Bohrer zur zweckmäßigen Befreiung des Hülseninneren geeignet ist. Hierdurch ist jedoch eine sehr exakte Ausrichtung des Bohrers erforderlich, um das Hülseninnere so zu treffen, dass der Bohrvorgang vollen Erfolg hat. Durch die schlechten Transparenzeigenschaften von Sporteis ist die Hülse unter der Eisfläche mit bloßem Auge jedoch nur sehr schlecht erkennbar, sodass mehrere Ansätze und sukzessive Bohrvorgänge erforderlich werden, wenn man dabei verhindern möchte, dass versehentlich die Hülse selbst angebohrt und beschädigt wird (vgl. Figur 3).

Die zahlreichen Bohransätze und Bohrvorgänge sorgen für Frustrationen, ferner geht wertvolle Arbeitszeit verloren. Die Hülsen werden beschädigt, da sie versehentlich angebohrt werden, und verschleißen stark, sodass sie häufiger getauscht werden müssen.

Zudem wird die Eisfläche und insbesondere der Sitz der Hülse in der Eisfläche destabilisiert, wenn das Eis mehrfach angebohrt werden muss.

Der Erfolg beim Anbohren ist dabei in der Praxis stark dem Zufall überlassen.

Ein ähnliches Problem stellt sich regelmäßig in der Bau- und Gebäudetechnik. Beispielsweise werden wichtige Elemente in Unterputzdosen hinterlegt und sodann verputzt. Hier steht man regelmäßig vor der Schwierigkeit, diese Unterputzdosen wieder aufzufinden und im Raum (Ort, Ausrichtung, etc.) genauer verorten zu können, z.B. zwecks kontrolliertem/gezieltem Zugang oder (u.a. auch teilweiser) Freilegung, z.B. zwecks Reparatur, Wartung oder Erweiterung.

Eine bekannte Vorgehensweise besteht darin, auf eine Dokumentation der Verortung (beispielsweise der Unterputzdosen) zurückzugreifen. Diese ist jedoch häufig veraltet, nicht genau genug oder aus anderen Gründen falsch. Auch können hinterlassene Markierungen (z.B. Striche an der Wand) als Indikator genutzt werden. Noch eine weitere Vorgehensweise besteht in der logischen und/oder psychologischen Deduktion ("wenn ich es installiert/eingebracht hätte, wäre es irgendwo hier"). Häufig wird durch diese bekannten Techniken zunächst eine Groblokalisation der aufzufindenden Komponente erreicht. Danach wird durch Ausführen von Versuchen in multiplen Anläufen ("trial and error", d.h. Ausprobieren) die Oberfläche, in der Regel also mehrfach, durchbrochen/aufgetan, bis das Ziel gefunden ist.

Aus US2012/289365A1 ist mit dem Goal Post Retention System ein Torpfosten-Rückhaltesystem bekannt. Dabei kann ein ferromagnetischer Torpfosten eingesetzt werden, welcher dann sicher sitzt. Magnetstärken sind so optimiert, dass verhindert wird, dass der Torpfosten entfernt wird, ohne dass eine hinreichend hohe Kraft aufgewendet wird (preventing the goal post from becoming dislodged without application of sufficient force). Ferner wird eine verbesserte Eisfläche offenbart.

US6245001 B1 offenbart Trainingsgeräte zum Muskelaufbau von Athleten auf Basis von Ferromagneten. Hierdurch können Geschwindigkeit und Stärke eines "strokes" des Athleten in besonders angepasster Weise trainiert und verbessert werden.

Der vorliegenden Erfindung liegt die technische Aufgabe zugrunde, die Nachteile des Standes der Technik zu überwinden und Vorrichtungen und Verfahren zu schaffen, welche ein präzises Anbohren von beispielsweise Wänden oder Untergründen wie beispielsweise Eisflächen, insbesondere bereits im ersten Ansatz, ermöglichen.

Das Problem wird gelöst durch die beanspruchten, miteinander in Beziehung stehenden Verfahren und Vorrichtungen.

Insbesondere wird eine allgemeine "Suchmaus" geschaffen, welche das Problem im Bausektor löst, aber auch in anderen Anwendungsfeldern vorteilhaft eingesetzt werden kann. Zudem wird eine "Eismaus" geschaffen, welche konkret das Problem mit den erneuerten Eisflächen, beispielsweise im Eissport, löst.

### Beschreibung der Erfindung

Die vorliegende Erfindung schafft ein Verfahren mit den Merkmalen des Anspruchs 1. Weitere vorteilhafte Ausgestaltungen sind in den Unteransprüchen angegeben.

Es ist ein Verfahren zum Lokalisieren einer aufzufindenden Komponente, insbesondere unter einer Oberfläche und/oder einer Abdeckung, insbesondere unter einer intransparenten und/oder nur schlecht transparenten Oberfläche und/oder Abdeckung, vorgesehen. Das Verfahren kann einen Schritt eines Bereitstellens einer Auffindevorrichtung umfassen. Diese kann geeignet sein, mit einem Magnetfeld mindestens eines Magneten, insbesondere eines Permanentmagneten, insbesondere Permanentmagneten, welcher an oder unter einer Oberfläche und/oder Abdeckung angeordnet ist, zu interagieren. Hierdurch kann so beispielsweise auf einen Ort und/oder eine Ausrichtung des mindestens einen Magneten geschlossen werden. Das Verfahren kann einen Schritt eines Bewegens der Auffindevorrichtung, insbesondere unter Ausrichten der Auffindevorrichtung, in ein oder einem Magnetfeld, umfassen. Das Magnetfeld kann dabei von einer aufzufindenden Komponente bereitgestellt werden. Das Magnetfeld kann auch, zusätzlich oder exklusiv, von mindestens einem an oder im Bereich einer aufzufindenden Komponente angeordneten Magneten, insbesondere Permanentmagneten, bereitgestellt werden.

Das Verfahren ist sehr vorteilhaft, was am Anwendungsbeispiel des Bausektors kurz erläutert sei. Eine Unterputzdose ist beispielsweise eine aufzufindende Komponente. Durch einen hieran angeordneten Magneten (oder anderweitig erzeugte magnetische Eigenschaften) wird die Unterputzdose später leicht und präzise aufgefunden, ohne dass eine Oberfläche aufgebohrt oder freigelegt werden muss. In einigen Ausführungsformen kann zudem präzise auf eine geometrische Ausrichtung der Unterputzdose geschlossen werden. So kann zu der Unterputzdose ein präziser Zugang geschaffen werden und/oder beispielsweise die Unterputzdose freigelegt werden, wobei weder die Unterputzdose noch die intakte verputzte Oberfläche andernorts zerstört wird oder angegriffen werden muss.

Es kann auf direktem als auch auf indirektem oder implizitem Wege auf den Ort und/oder die Ausrichtung der Komponente geschlossen werden. Beispielsweise wird ein Magnetfeld von der Komponente selbst erzeugt, oder eine nicht oder nur schwach magnetische Komponente wird zum Zwecke des Erfindungsgebrauchs mit einem oder mehreren Magneten ausgestattet.

Eine schlecht transparente oder semitransparente Oberfläche ist beispielsweise Eis. Eine Putzschicht an einer Wand ist regelmäßig intransparent.

So können auch unter Eisflächen verborgene Komponenten (Gegenstände) präzise lokalisiert und ggf. zerstörungsfrei freigelegt werden. Dies ist beispielsweise bei Eishockeytoren sehr sinnvoll. Hierfür müssen regelmäßig Aufnahmehülsen der Eishockeytore freigelegt werden, z.B. durch Bohren, nachdem eine Eissportfläche erneuert wurde.

Eine Auffindevorrichtung kann beispielsweise ein Magnetometer umfassen, z.B auch mittels einer Applikation für Mobiltelefone. In einem einfachen Falle ist jedoch ein Magnet, z.B. ein Stabmagnet, an einer beweglichen Aufhängung aufgehängt. Das zweite Ende der Aufhängung kann an einem Stativ befestigt sein. Das gibt dem Nutzer wertvolle Hilfestellung beim genauen Ausrichten/Verorten und ggf. beim Markieren der gefundenen Stelle (z.B. zum Aufbohren oder anderweitig Freilegen). Auffindevorrichtungen können beispielsweise mechanisch ausschlagen, oder auch durch eine graphische Darstellung, z.B. auf dem Display eines Mobiltelefons. Auf einem Display können auch weitere Angaben dargestellt sein, z.B. der Ort der aufzufindenden Vorrichtung und/oder eine Winkelangabe. Diese Angaben können beispielsweise für ein präzises Bohren und/oder anderweitiges Freilegen genutzt werden. Ein "signalgebendes Ausschlagen" ist beispielsweise in Rahmen einer Ausführungsform gegeben, welche eine solche Applikation für ein Mobiltelefon umfasst. Im Wesentlichen wird hierbei also ein Magnetfeld gemessen, und in irgendeiner weiterverwendbaren Form zugänglich gemacht. Beispielsweise wird ein Messergebnis, in irgendeiner geeigneten Form, graphisch dargestellt und beispielsweise über ein Display einem Nutzer graphisch oder auch beispielsweise über akustische Signale zugänglich gemacht. Es können auch graphische und akustische Signale kombiniert werden. In einem Beispiel wird graphisch eine Korrekturrichtung und akustisch eine verbleibende Korrekturdistanz indiziert.

Ein Bewegen in einem Magnetfeld kann unterschiedlichste Formen, Arten und Weisen von Bewegungen umfassen. Besonders angedacht ist, dass eine Auffindevorrichtung in einen Kernbereich eines Magnetfeldes eingebracht wird. Ein Kernbereich kan beispielsweise ein Bereich sein, wo ein Magnetfeld eine gewisse, nicht zu vernachlässigende Stärke hat und/oder beispielsweise (z.B. von der Auffindevorrichtung) messbar ist. Verschiedene Bewegungen sind grundsätzlich denkbar, und können beispielsweise auch Bewegungsformen wie Annähern, Heranführen, Bewegen in oder um etwas herum, umfassen.

In einem Beispiel ist ein Magnetfeld ein Feld, welches mit r⁻² abfällt. Ein Beispiel für einen Kernbereich kann beispielsweise ein Bereich sein, wo die Magnetfeldstärke noch 10%, 5%, 2% oder 1 % einer in unmittelbarer Nähe zum Magneten gemessenen Magnetfeldstärke hat. Dies kann zudem abhängig sein, u.a. von der Genauigkeit und Sensibilität der Auffindevorrichtung, welche zum Einsatz gebracht wird.

Bei den Auffindevorrichtungen können, insbesondere bei der mechanischen Variante, bestimmte weitere Faktoren eine tatsächliche Ausrichtung am Magnetfeld beeinflussen. Zeigt eine mechanische Vorrichtung beispielsweise zur Seite, wird also ausgelenkt und/oder schlägt aus, so kann ein Einfluss von Schwerkraft bewirken, dass Ausschlag und Magnetfeld sich nicht 100% entsprechen bzw. parallel verlaufen. Ungeachtet dessen kann mit der Auffindevorrichtung ein präziser Punkt gefunden werden, wo die Magnetfeldlinien senkrecht verlaufen, und daher beispielsweise kein Ausschlag stattfindet. Hierzu wird beispielsweise die Auffindevorrichtung in Richtung des angezeigten Ausschlags im bzw. durch das Magnetfeld bewegt, bis z.B. der Ausschlag verschwindet/gegen Null geht. So wird beispielsweise die Auffindevorrichtung ausgerichtet, beispielsweise durch den Nutzer. Der Nutzer führt dieses Ausrichten der Auffindevorrichtung beispielsweise fort, bis er den koaxialen Punkt gefunden hat. Ein solches Ausrichten kann an oder auf der Oberfläche stattfinden. In einem Beispiel kann eine Auffindevorrichtung dabei auf der Oberfläche mechanisch aufliegen, beispielsweise auch durch ein Stativ. Ein Ausrichten kann dann ein Schieben und/oder Verschieben der Auffindevorrichtung über die Oberfläche beinhalten.

Ein anderes Beispiel für eine Auffindevorrichtung basiert auf magnetischen und/oder magnetsensitiven Kugeln. Diese können sich beispielsweise in einer Vorrichtung frei, oder jedenfalls in bestimmten Richtungen frei, bewegen. Aus dem Verhalten der Kugeln kann beispielsweise auf den Ort der aufzufindenden Komponente geschlossen werden. Hier kann eine Ausrichtung der Vorrichtung im Schwerefeld vonnöten bzw. vorteilhaft sein. Hierdurch werden Fehler vermieden, welche ansonsten dadurch auftreten, dass sich die Kugeln im Schwerefeld ausrichten, was sich mit der Ausrichtung im Magnetfeld überlagert, und hierdurch Fehler verursacht. Beispielsweise können Prüfgeräte (z.B. "Wasserwaage") und Einstellmechanismen (z.B. Stellschrauben) vorgesehen sein. Ein "Prüfgerät zur horizontalen oder vertikalen Ausrichtung" ist dabei beispielsweise eine einfache "Wasserwaage" ("horizontale und vertikale Ausrichtung" sind dabei lediglich als abstrakte und beschreibende Eignungsmerkmale für ein solches Prüfgerät zu verstehen, es reicht jedoch aus, wenn ein solches Prüfgerät für lediglich eine der genannten Ausrichtungen zum Einsatz kommt). In einem Beispiel sind zwei Prüfgeräte vorgesehen und drei Stellschrauben. Hierdurch kann die Vorrichtung exakt senkrecht (in x- und y-Ebene) zum Schwerefeld (entlang der z-Achse) ausgerichtet werden. So funktioniert die Bestimmung des Ortes und/oder der Ausrichtung der aufzufindenden Komponente auch dann exakt, wenn die Bodenoberfläche beispielsweise nicht exakt planar und/oder selbst nicht exakt senkrecht zum Schwerefeld ist.

Ein weiteres Beispiel ist ein Prüfgerät, welches "von sich aus" in zwei Dimensionen die Ausrichtung prüft (bspw. "zweidimensionale Wasserwaage").

Um deren Auffindbarkeit zu ermöglichen, werden beispielsweise Unterputzdosen oder Aufnahmehülsen für den Eissport mit Magneten ausgestattet. Alternativ und/oder zusätzlich können auch die Aufnahmehülsen selbst magnetisiert werden. Idealerweise erfolgt die Ausstattung und damit verbundene Anordnung so, dass ein Bezugspunkt, welcher später wieder aufgefunden werden soll, durch senkrecht austretende magnetische Feldlinien markiert wird. Beispielsweise kann der Bezugspunkt auf einer magnetischen Feldlinie liegen, welcher eine Gerade beschreibt.

Das Feld kann von einem Magneten erzeugt werden. Es können aber auch mehrere Magneten eingesetzt werden (Superpositionsprinzip). So können Bezugspunkte erzeugt werden, durch Überlagerung magnetischer Felder, ohne dass ein Magnet selbst notwendigerweise im Bereich des Bezugspunktes eingebracht werden muss (beispielsweise ist letzteres aufgrund der Beschaffenheit der aufzufindenden Komponente selbst nicht möglich). Insbesondere können zwei oder vier Magnete vorgesehen sein. Insbesondere sind derartige Superpositionen beim Einsatz einzelner Würfelmagnete vorteilhaft. So können verstärkte Felder, aber insbesondere auch Dipolfelder, Quadrupolfelder (oder auch höhere Entwicklungsordnungen) zum Markieren von Bezugspunkten eingesetzt werden.

In einer medizintechnischen Anwendung werden beispielsweise ein Anfang und ein Ende eines Objektes (beispielsweise eine medizinische Schraube) mit jeweils einem (oder mehreren) Magneten ausgestattet. Hierdurch können teure und aufwändige (und teilweise schädliche) medizinische Untersuchungen (z.B. Röntgenbilder) durch den Einsatz der vorliegenden Erfindung vermieden werden.

Für ein verbessertes beispielhaftes Verständnis der Ausführungsformen wird auf die Figuren und die zugehörigen Figurenbeschreibungen verwiesen.

Sämtliche im Zusammenhang mit entsprechenden Verfahren offenbarte Merkmale können im Zusammenhang mit den Vorrichtungen zum Einsatz gebracht werden, als auch umgekehrt. Insbesondere sind auch Merkmale der Suchmaus mit den Merkmalen der Eismaus kompatibel, und umgekehrt.

### Figurenliste

Die vorliegende Erfindung wird nachfolgend anhand der in den schematischen Figuren der Zeichnungen angegebenen Ausführungsbeispiele näher erläutert. Es zeigen dabei:
- Fig. 1: eine Ausführungsform der vorliegenden Erfindung;
- Fig. 2: eine zweite Ausführungsform der vorliegenden Erfindung (insbesondere Eismaus);
- Fig. 3: eine Illustration eines Problems im Stand der Technik;
- Fig. 4: eine Ausführungsform der vorliegenden Erfindung (insbesondere Eismaus).

In allen Figuren sind gleiche bzw. funktionsgleiche Elemente und Vorrichtungen - sofern nichts anderes angegeben ist - mit denselben Bezugszeichen versehen worden.

### Beschreibung der Zeichnungen

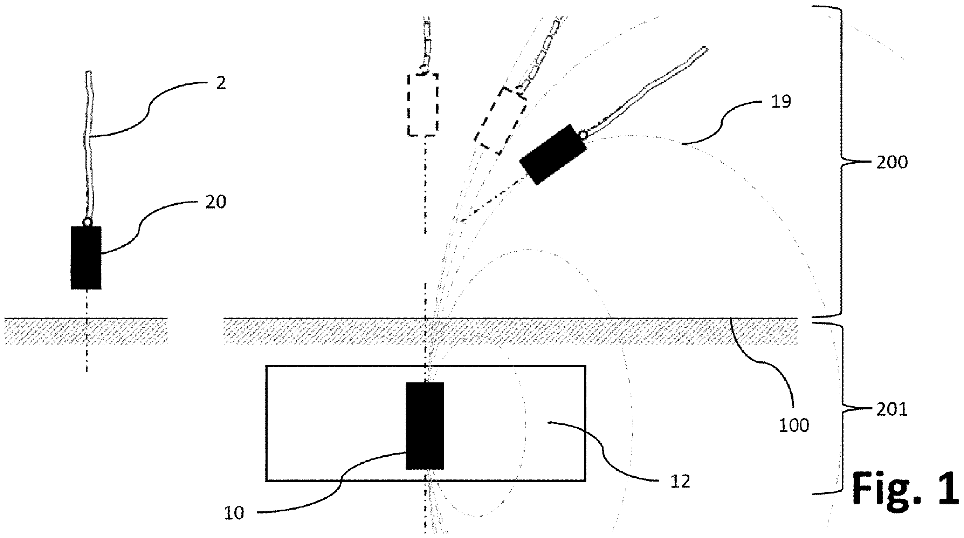

Die Figur 1 zeigt eine mögliche Ausführungsform der vorliegenden Erfindung. Es ist ein Bereich 200 oberhalb einer Oberfläche 100 und ein Bereich 201 unterhalb einer Oberfläche 100 dargestellt. Es kann sich beispielsweise um eine Wand, eine Decke oder einen Boden handeln (Unterputzdose 12 mit Magnet 10). Im vorliegenden Beispiel ist insbesondere auch eine Eisfläche 100 denkbar. Die aufzufindende Komponente 12 ist dann im Eis eingebracht. Mit einem Magnetometer kann die aufzufindende Komponente lokalisiert werden. Es ist auch möglich, eine Ausrichtung der Komponente genauer zu bestimmen. Beispielsweise wird ein Mobiltelefon durch das Magnetfeld 19 bewegt. In einem Beispiel werden dabei mehrere (zwei oder mehr) Messungen, insbesondere an unterschiedlichen Orten aufgenommen. Hierdurch sind die Rückschlüsse auf die Ausrichtung besonders präzise.

In einem einfachen Beispiel kommt ein Magnet 20 an einer Aufhängung 2 zum Einsatz. Der Magnet 20 kann sich also an der Aufhängung frei bewegen und sich im Magnetfeld 19 ausrichten. Ein hinreichend leichter Magnet 20 kann hierbei vorteilhaft sein. Durch Bewegen des aufgehängten Magneten 20 können so ein Ort und eine Ausrichtung des Magneten 10 genauer bestimmt werden. Hierbei bleibt die Oberfläche 100 völlig intakt. Der Magnet 20 kann ein Stabmagnet sein. Es können aber auch komplexere Strukturen (z.B. ein Tellermagnet, oder mehrere kleine Magneten), zum Einsatz gebracht werden. Hierdurch kann besonders präzise (und gar simultan) auf Ort und Ausrichtung des Magneten 10 geschlossen werden. Beispielsweise ergibt sich dies aus einer Auslenkung der Aufhängung 2 und einer Ausrichtung des/der Magneten 20 im Raum (zwei Winkel). In einem weiteren Beispiel wird das Gewicht des Magneten hierzu benutzt, denn dies hat eine Auswirkung auf die Winkelauslenkung der Aufhängung.

In einem einfachen Beispiel kommt lediglich ein Stabmagnet zum Einsatz. Beispielsweise wird dieser bewegt, durch Bewegung der Aufhängung, bis die Ausrichtung des Magneten 20 samt Aufhängung lotrecht zur Oberfläche 100 ist. Die so gefundene Stelle auf der Oberfläche kennzeichnet die Stelle, unter welcher sich der Magnet 10 befindet.

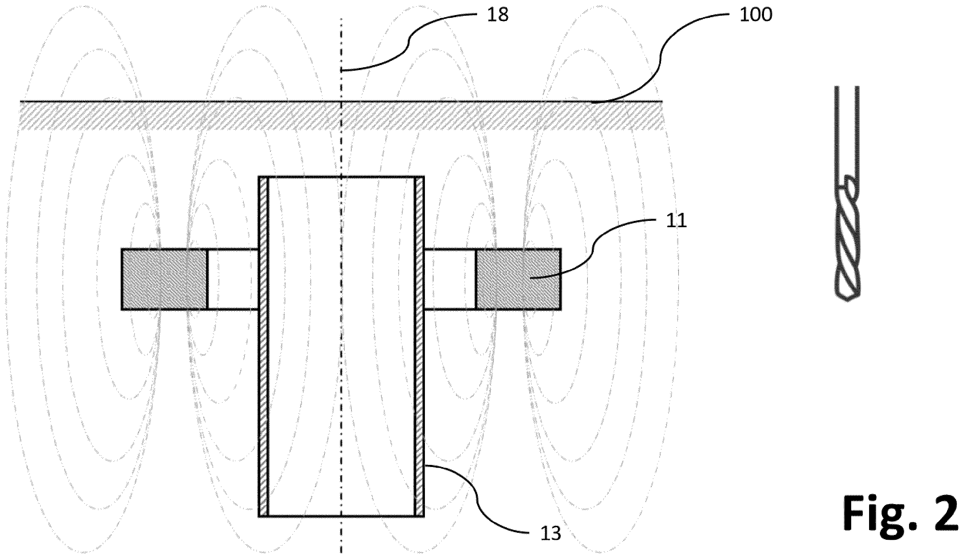

Die Figur 2 zeigt eine zweite mögliche Ausführungsform der vorliegenden Erfindung (insbesondere geeignet für die "Eismaus"). Daher sei diese Ausführungsform anhand des Eisbeispiels beschrieben. Unter einer erneuerten Eisfläche 100 befindet sich eine Aufnahmehülse 13. Durch eine erfolgte Erneuerung des Eis ist diese Aufnahmehülse durch Eis verschlossen. Um beispielsweise ein Eishockeytor, oder eine andere Stabvorrichtung, aufzunehmen, soll diese Aufnahmehülse 13 im Innern, beispielsweise durch Anbohren, präzise freigelegt werden. Mittels der Erfindung wird nun eine Achse 18 für das präzise Anbohren bestimmt. Dies wird ermöglicht durch einen Ringmagneten oder Hohlmagneten 11, welcher ein entsprechendes Magnetfeld erzeugt. Der Ring- oder Hohlmagnet 11 umringt in diesem Beispiel die Hülse 13. Er kann aber auch beispielsweise anderweitig koaxial zur Hülse 13, z.B. höher oder tiefer im Eis, liegen als die Hülse 13 selbst. In einem weiteren Beispiel kann die Hülse 13 selbst magnetisch sein. Dann wird beispielsweise kein separater Magnet 11 benötigt.

Die Oberfläche 100 kann auch beispielsweise ein Boden oder eine Wand sein, die Anwendung der Erfindung ist nicht notwendigerweise auf den Fall der "Eismaus" beschränkt.

Die Figur 3 zeigt eine Illustration eines Problems im Stand der Technik. Ist, beispielsweise unter Eis mit schlechten Transparenzeigenschaften, der genaue Ort einer Aufnahmehülse nicht bekannt, kann es sein, dass man mehrfach bohren muss. Dabei destabilisiert man das frisch erneute Eis. Zudem ist eine Beschädigung der Hülse möglich, wenn man "fast genau", aber eben nicht "genau genug" das Ziel trifft. Dies ist häufig der Fall, wenn man versucht, durch "Hindurchgucken" durch das schlecht transparente Eis den Ort zum Aufbohren - also optisch - zu ermitteln.

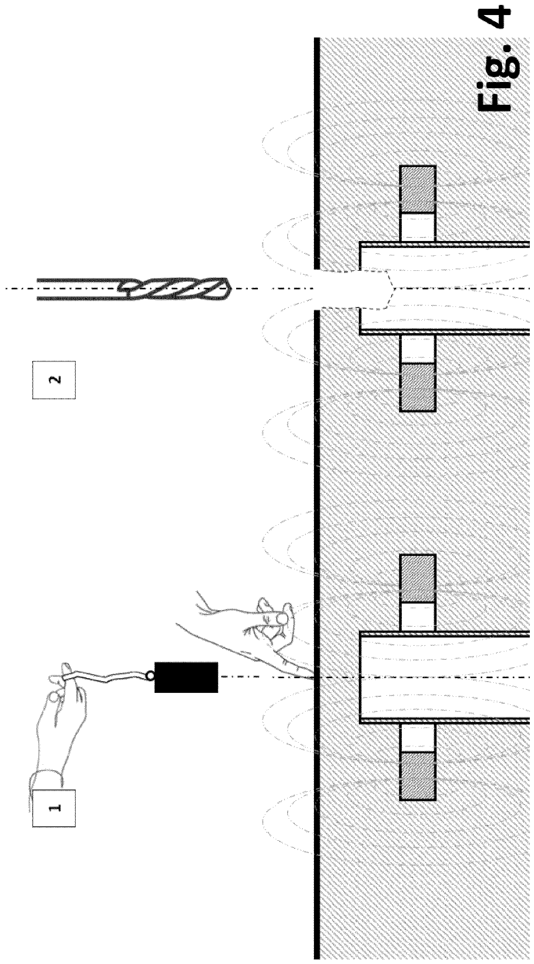

Die Figur 4 zeigt eine Ausführungsform der vorliegenden Erfindung (insbesondere geeignet für die "Eismaus").

In einem ersten Schritt wird eine Auffindevorrichtung in das Magnetfeld eingedrängt. Beispielsweise wird ein Magnetometer bewegt, welches beispielsweise auch Magnetfelddaten protokollieren kann (z.B. um hieraus eine Karte zu erstellen). In einem einfachen Beispiel genügt ein Magnet an einer flexiblen Aufhängung. Es kann auch beispielsweise ein Stativ vorgesehen sein: Dann kann der flexibel aufgehängte Magnet mittels des Stativs über die Oberfläche geschoben werden, um die genaue Bohrachse zu ermitteln. Andere Ausführungsformen sind denkbar. Beispielsweise sind auch kugelbasierte magnetische Vorrichtungen denkbar (siehe allgemeine Erfindungsbeschreibung), aus deren Roll- und Ausrichtungsverhalten auf die Bohrachse geschlossen werden kann (zur Ausrichtung einer solchen Vorrichtung im Schwerefeld kann dabei eine Wasserwaage oder dergleichen sachdienlich sein).

In einem zweiten Schritt wird präzise entlang der Achse gebohrt. Hier wird das Innere der Hülse freigelegt. Sodann kann ein Stab, z.B. eine Flagge oder ein Eishockeytor, in die freigelegte Aufnahmehülse eingeführt werden, und steckt sodann sicher in einer anderweitig unversehrten und intakten Eisfläche.

### Weitere Offenbarungen (Suchmaus)

### Merkmale auch für Eismaus geeignet

1. Verfahren zum Lokalisieren einer aufzufindenden Komponente (12, 13), insbesondere unter einer Oberfläche und/oder einer Abdeckung (100), insbesondere unter einer intransparenten und/oder nur schlecht transparenten Oberfläche und/oder Abdeckung, umfassend die folgenden Schritte:
   - Bereitstellen einer Auffindevorrichtung (2, 20), welche geeignet ist, mit einem Magnetfeld mindestens eines Magneten (10, 11), insbesondere eines Permanentmagneten, insbesondere Permanentmagneten, welcher an oder unter einer Oberfläche und/oder Abdeckung (100) angeordnet ist, derart zu interagieren, dass auf einen Ort und/oder eine Ausrichtung des mindestens einen Magneten (10, 11) geschlossen werden kann;
   - Bewegen der Auffindevorrichtung (2, 20), insbesondere unter Ausrichten der Auffindevorrichtung, in einem Magnetfeld (19), welches von einer aufzufindenden Komponente (12, 13) bereitgestellt wird und/oder von mindestens einem an oder im Bereich einer aufzufindenden Komponente (12, 13) angeordneten Magneten (10, 11), insbesondere Permanentmagneten, bereitgestellt wird.
2. Verfahren nach Offenbarung 1, welches, insbesondere im Anwendungskontext, einen logischen Schluss auf den Ort und/oder die Ausrichtung des Magneten (10, 11), insbesondere an oder unter der Oberfläche und/oder Abdeckung (100), und/oder auf den Ort und/oder die Ausrichtung der aufzufindenden Komponente (12, 13), ermöglicht.
3. Verfahren nach Offenbarung 1 oder 2, wobei das Bewegen der Auffindevorrichtung (2, 20) ein Ausschlagen der Auffindevorrichtung, insbesondere mechanisches Ausrichten am Magnetfeld (19), insbesondere auch signalgebendes Ausschlagen, insbesondere an einer Oberfläche (100), insbesondere koaxial am Magnetfeld (19), umfasst, wobei mit der Auffindevorrichtung ein Ort, eine Ausrichtung und/oder eine Konfiguration der Auffindevorrichtung erreicht wird, bei dem/der/denen sich magnetische Feldlinien des mindestens eines Magneten im Wesentlichen senkrecht, insbesondere zwischen 80 und 100 Grad, bevorzugt zwischen 85 und 95 Grad, in Bezug auf die Oberfläche (100) verhalten.
4. Verfahren nach einer der vorhergehenden Offenbarungen, wobei das Bewegen der Auffindevorrichtung (2, 20) ein Ausschlagen, insbesondere mechanisches Ausrichten, insbesondere signalgebendes Ausschlagen, der Auffindevorrichtung, insbesondere an einer Oberfläche (100), insbesondere koaxial am Magnetfeld (19), umfasst, wobei ein Ort und/oder eine Ausrichtung der Auffindevorrichtung erreicht wird, bei dem/der/denen magnetische Feldlinien des mindestens eines Magneten (10, 11) im Wesentlichen senkrecht, insbesondere zwischen 80 und 100 Grad, bevorzugt zwischen 85 und 95 Grad, aus der Oberfläche (100) austreten und/oder durch die Oberfläche (100) hindurchtreten.
5. Verfahren nach einer der vorhergehenden Offenbarungen, wobei die aufzufindende Komponente (12, 13) eine Unterputzdose und/oder ihren Inhalt und/oder einer Unterputzdose beigeordnete Komponenten umfasst.
6. Verfahren nach einer der vorhergehenden Offenbarungen, wobei die Oberfläche eine Bodenfläche (100) und/oder Eisfläche (100) umfasst, und wobei die aufzufindende Komponente eine Aufnahmevorrichtung (13) für einen im Boden und/oder im Eis aufzunehmenden oder zu befestigenden Gegenstand, wie beispielsweise Sport-Torvorrichtungen, wie beispielsweise Wintersporttorvorrichtungen, wie beispielsweise Eissporttore, wie beispielsweise Eishockeytore, umfasst.
7. Verfahren nach einer der vorhergehenden Offenbarungen, wobei die Auffindevorrichtung (2, 20)
   ein Pendel (2) und/oder eine flexible Aufhängung (2) mit einer daran befindlichen magnetischen Komponente (20), insbesondere Stabmagnet (20), und/oder
   ein Magnetometer und/oder ein Endgerät, beispielsweise ein Mobiltelefon, umfasst.
8. Verfahren nach einer der vorhergehenden Offenbarungen, wobei die Auffindevorrichtung (2, 20) ein Stativ umfasst.
9. Verfahren nach einer der vorhergehenden Offenbarungen, wobei die Auffindevorrichtung (2, 20) magnetische und/oder magnetsensitive Kugeln und/oder Bälle umfasst, insbesondere Kugeln und/oder Bälle, welche mindestens einen Bewegungsfreiheitsgrad aufweisen.
10. Verfahren nach Offenbarung 9, wobei die Auffindevorrichtung (2, 20) ferner ein Prüfgerät zur horizontalen oder vertikalen Ausrichtung, insbesondere eine Wasserwaage, aufweist, insbesondere ferner eine zusätzliche Fläche für Kugeln und/oder Bälle aufweist, welche mit dem Prüfgerät im Schwerefeld ausgerichtet werden kann.
11. Auffindevorrichtung (2, 20) zum Lokalisieren einer aufzufindenden Komponente (12, 13), insbesondere unter einer Oberfläche und/oder einer Abdeckung (100), insbesondere unter einer intransparenten und/oder nur schlecht transparenten Oberfläche und/oder Abdeckung, wobei die Auffindevorrichtung (2, 20) geeignet ist, mit einem Magnetfeld mindestens eines Magneten (10, 11), insbesondere eines Permanentmagneten, insbesondere Permanentmagneten, welcher an oder unter einer Oberfläche und/oder Abdeckung (100) angeordnet ist, derart zu interagieren, dass auf einen Ort und/oder eine Ausrichtung des mindestens einen Magneten geschlossen werden kann, und welche dazu geeignet ist, insbesondere unter Ausrichten der Auffindevorrichtung (2, 20) in einem Magnetfeld (19), den Ort und/oder eine Ausrichtung einer aufzufindenden Komponente (12, 13) mittels des Magnetfeldes (19), welches von der aufzufindenden Komponente (12, 13) bereitgestellt wird und/oder von mindestens einem an oder im Bereich der aufzufindenden Komponente (12, 13) angeordneten Magneten (10, 11), insbesondere Permanentmagneten, bereitgestellt wird, zu bestimmen.
12. Auffindevorrichtung (2, 20) nach Offenbarung 11, umfassend eine flexible Aufhängung, Pendel- und/oder Fadenaufhängung (2) mit einer daran befindlichen magnetischen Komponente (20), und/oder ein Magnetometer, insbesondere ferner umfassend ein Stativ, welches ein kontrolliertes Ausrichten der Auffindevorrichtung (2, 20), insbesondere auch ein freies Schwingen der flexiblen Aufhängung, Pendel- und/oder Fadenaufhängung (2), ermöglicht.
13. Auffindevorrichtung (2, 20) nach Offenbarung 11 oder 12, umfassend mindestens einen magnetischen und/oder magnetsensitive/n Kugel und/oder Ball, insbesondere mehrere magnetische und/oder magnetsensitive Kugeln und/oder Bälle, insbesondere Kugel/n und/oder Ball/Bälle, welche mindestens einen Bewegungsfreiheitsgrad aufweisen, insbesondere wobei durch ein Rollen der Kugel/n und/oder des Balles/der Bälle auf einen Ort und/oder eine Ausrichtung des mindestens einen Magneten (10, 11) geschlossen werden kann.
14. Auffindevorrichtung (2, 20) nach einer der Offenbarungen 11 - 13, wobei die Auffindevorrichtung (2, 20) ferner ein Prüfgerät zur horizontalen und/oder vertikalen Ausrichtung, insbesondere eine Wasserwaage, aufweist, insbesondere ferner eine zusätzliche Fläche für Kugeln und/oder Bälle aufweist, welche mit dem Prüfgerät im Schwerefeld ausgerichtet werden kann.
15. Auffindbare Vorrichtung (10 - 13) zur verbesserten Lokalisierbarkeit von einer später aufzufindenden Komponente (12, 13), insbesondere unter einer Oberfläche (100) und/oder einer Abdeckung, insbesondere unter einer intransparenten und/oder nur schlecht transparenten Oberfläche und/oder Abdeckung, wobei die Vorrichtung eine aufzufindende Komponente (12, 13) und mindestens einen Magneten (10, 11) umfasst, welcher derart in Bezug auf die aufzufindende Komponente (12, 13) angeordnet und/oder ausgerichtet ist, dass mittels einer magnetsentitiven Auffindevorrichtung (2, 20), insbesondere einer Auffindevorrichtung nach einer der Offenbarungen 11 - 14, Rückschlüsse auf Ort und/oder räumliche Ausrichtung der aufzufindenden Komponente (12, 13) gezogen werden können, ohne eine eventuelle Oberfläche (100), hinter welcher die aufzufindende Komponente (12, 13) verborgen sein kann, zerstören, öffnen und/oder aufbrechen zu müssen.
16. Auffindbare Vorrichtung (10 - 13) nach Offenbarung 15, insbesondere Unterputzdose, welche unter einer Oberfläche und/oder einer Abdeckung (100), insbesondere unter einer intransparenten und/oder nur schlecht transparenten Oberfläche und/oder Abdeckung, angeordnet und/oder von dieser verdeckt ist, insbesondere dort verputzt ist.
17. Auffindbare Vorrichtung (10 - 13) nach Offenbarung 15 oder 16, wobei das Magnetfeld (19) des mindestens einen Magneten (10, 11) derart beschaffen ist, dass die Magnetfeldlinien eine Oberfläche und/oder Abdeckung (100) im Wesentlichen senkrecht, insbesondere zwischen 80 und 100 Grad, bevorzugt zwischen 85 und 95 Grad, durchdringen, insbesondere durch Einsatz von ein oder mehreren Ringmagneten (11), Hohlmagneten (11) und/oder einem oder mehreren Würfelmagneten.
18. Auffindbare Vorrichtung (10 - 13) nach einer der Offenbarungen 15 - 17, umfassend eine Aufnahmevorrichtung (13) für einen im Boden (201) und/oder im Eis (201) aufzunehmenden oder zu befestigenden Gegenstand, wie beispielsweise Sport-Torvorrichtungen, wie beispielsweise Wintersporttorvorrichtungen, wie beispielsweise Eissporttore, wie beispielsweise Eishockeytore, geeignet zum Einbringen unterhalb einer Eisoberfläche (100).
19. Auffindbare Vorrichtung (10 - 13) nach einer der Offenbarungen 15 - 18, wobei mindestens zwei Magnete (10), insbesondere Würfelmagnete, insbesondere an den Enden in den Endbereichen einer aufzufindenden Komponente (12, 13), eingesetzt werden.
20. Verfahren zum Einrichten verbesserter Lokalisierbarkeit einer Komponente (12, 13), insbesondere unter einer Oberfläche und/oder einer Abdeckung (100), insbesondere unter einer intransparenten und/oder nur schlecht transparenten Oberfläche und/oder Abdeckung aufzufindenden Komponente (12, 13), umfassend:
   Bereitstellen einer Komponente (12, 13), welche geeignet ist, unter einer Oberfläche oder Abdeckung verdeckt zu werden, insbesondere verputzt, in den Boden eingebracht und/oder in Eis eingebracht zu werden;
   Anordnen mindestens eines Magneten (10, 11) an der Komponente, welcher derart in Bezug auf die Komponente angeordnet und/oder ausgerichtet ist, dass mittels einer magnetsentitiven Auffindevorrichtung (2, 20), insbesondere einer Auffindevorrichtung nach einer der Offenbarungen 11 - 14, Rückschlüsse auf Ort und/oder räumlicher Ausrichtung der aufzufindenden Komponente (12, 13) gezogen werden können, ohne eine eventuelle Oberfläche (100), hinter welcher die aufzufindende Komponente (12, 13) verborgen, insbesondere verputzt, sein kann, öffnen, zerstören und/oder aufbrechen zu müssen.
21. Verfahren nach Offenbarung 20, ferner umfassend: Verputzen der Komponente (12, 13), insbesondere umfassend eine Unterputzdose (12), insbesondere unter Zuhilfenahme von Putz.
22. Set, umfassend die Auffindevorrichtung (2, 20) nach einer der Offenbarungen 11 - 14 sowie eine oder mehrere Vorrichtungen (10 - 13) zur verbesserten Lokalisierbarkeit nach einer der Offenbarungen 15 - 19 und/oder einen oder mehrere Magneten.
23. Verwendung einer Vorrichtung (10 - 13) nach einer der Offenbarungen 11 - 19 oder 22 und/oder Nutzung eines Verfahrens nach einer der Offenbarungen 1 - 10, 20 oder 21 im Bausektor, insbesondere beim Installieren, Verputzen und/oder Auffinden von Komponenten, deren spätere Wiederauffindung und Lagebestimmung vorteilhaft sein kann.
24. Verwendung einer Vorrichtung (2, 20, 10 - 13) nach einer der Offenbarungen 11 - 19 oder 22 und/oder Nutzung eines Verfahrens nach einer der Offenbarungen 1 - 10, 20 oder 21 im Eissport-, Niedertemperatursport- und/oder Wintersportanlage, insbesondere beim Installieren, Eisüberdecken und/oder Auffinden von Komponenten, deren spätere Wiederauffindung und Lagebestimmung vorteilhaft sein kann, im Eis.
25. Verwendung und/oder Nutzung nach Offenbarung 24, wobei mindestens eine Komponente (12, 13) eine Aufnahmehülse (13) für Stäbe und/oder stabartige Teile, insbesondere Aufnahmehülsen (13) für Füße von Eissporttoranlagen, umfasst.
26. Verwendung einer Vorrichtung (2, 20, 10 - 13) nach einer der Offenbarungen 11 - 19 oder 22 und/oder Nutzung eines Verfahren nach einer der Offenbarungen 1 - 10, 20 oder 21, insbesondere Verwendung der Vorrichtung nach Offenbarung 19, in der Medizintechnik.

### Bezugszeichenliste

- 2: Faden/Pendel/Aufhängung
- 10: Magnet
- 11: Magnet (Ringmagnet)
- 12: aufzufindende Komponente (schematisch)
- 13: aufzufindende Komponente bspw. Aufnahmehülse für Stab (im Eis)
- 18: Achse
- 19: Magnetfeld
- 20: Stabmagnet
- 100: Oberfläche/Boden/Wand/Eisfläche
- 200: oberhalb Oberfläche
- 201: unter der Oberfläche

## Patentansprüche

1. Verfahren zum Lokalisieren einer aufzufindenden Komponente (12, 13), insbesondere eissportrelevante Befestigungskomponente, unter einer Eissportfläche umfassend die folgenden Schritte:
- Bereitstellen einer Auffindevorrichtung (2, 20), welche geeignet ist, mit einem Magnetfeld (19) mindestens eines Magneten (10, 11), insbesondere eines Permanentmagneten, insbesondere Permanentmagneten, welcher unter der Eissportfläche (100) angeordnet ist, derart zu interagieren, dass auf einen Ort und/oder eine Ausrichtung des mindestens einen Magneten (10, 11) geschlossen werden kann;
- Bewegen der Auffindevorrichtung (2, 20), insbesondere unter Ausrichten der Auffindevorrichtung, in einem Magnetfeld (19), welches von einer aufzufindenden Komponente (12, 13) bereitgestellt wird und/oder von mindestens einem an oder im Bereich einer aufzufindenden Komponente (12, 13) angeordneten Magneten (10, 11), insbesondere Permanentmagneten, bereitgestellt wird.

2. Verfahren nach Anspruch 2, wobei die aufzufindende Komponente (12, 13) eine Aufnahmehülse (13) für Stäbe und/oder stabartige Teile, insbesondere für Füße von Eissporttoranlagen, umfasst.

3. Verfahren nach Anspruch 3, wobei die Aufnahmehülse (13) das Magnetfeld (19) bereitstellt und/oder das Magnetfeld (19) durch mindestens einen Magneten (10, 11), insbesondere Ring- und/oder Hohlmagneten, welcher bei, an, in und/oder um die Aufnahmehülse (13) angeordnet ist, insbesondere in Bezug auf die Außenseite der Aufnahmehülse um die Aufnahmehülse herum und/oder im Wesentlichen koaxial zu dieser, insbesondere unter weniger als 10 Grad Abweichung, zu dieser angeordnet ist, bereitgestellt wird.

4. Verfahren nach einem der Ansprüche 1 - 3, ferner umfassend einen Schritt eines Freilegens eines Bereiches der eissportrelevanten Befestigungskomponente (13), insbesondere eines Freilegens eines Innenbereiches einer Aufnahmehülse (13), mit einem Bohrgerät, insbesondere Eisbohrer, und/oder einen Schritt eines Markierens, inbesondere zwecks späterer Freilegung, unter Nutzung der gewonnenen Erkenntnisse in Bezug auf deren Position und/oder Ausrichtung.

5. Verfahren nach einem der vorhergehenden Ansprüche, wobei die Auffindevorrichtung (2, 20) magnetische und/oder magnetsensitive Kugeln und/oder Bälle umfasst, insbesondere Kugeln und/oder Bälle, welche mindestens einen Bewegungsfreiheitsgrad aufweisen.

6. Verfahren nach Anspruch 5, wobei die Auffindevorrichtung (2, 20) ferner ein Prüfgerät zur horizontalen oder vertikalen Ausrichtung, insbesondere eine Wasserwaage, aufweist, insbesondere ferner eine zusätzliche Fläche für Kugeln und/oder Bälle aufweist, welche mit dem Prüfgerät im Schwerefeld ausgerichtet werden kann.

7. Auffindevorrichtung (2, 20) für den Winter- und Eissport zum Lokalisieren einer aufzufindenden Komponente (12, 13), insbesondere eissportrelevante Befestigungskomponente (13), unter einer Eissportfläche (100), wobei die Vorrichtung (2, 20) geeignet ist, mit einem Magnetfeld (19) mindestens eines Magneten (10, 11), insbesondere eines Permanentmagneten, insbesondere Permanentmagneten, welcher unter der Eissportfläche (100) angeordnet ist, derart zu interagieren, dass auf einen Ort und/oder eine Ausrichtung des mindestens einen Magneten (10, 11) geschlossen werden kann, wobei die Auffindevorrichtung (2, 20) ferner dazu geeignet ist, insbesondere unter Ausrichten der Auffindevorrichtung (2, 20) in einem Magnetfeld (19), mittels des Magnetfeldes (19), welches von einer aufzufindenden Komponente (12, 13) bereitgestellt wird und/oder von mindestens einem an oder im Bereich einer aufzufindenden Komponente (12, 13) angeordneten Magneten (10, 11), insbesondere Permanentmagneten, bereitgestellt wird, den Ort und/oder eine Ausrichtung der aufzufindenden Komponente (12, 13) zu bestimmen.

8. Auffindevorrichtung (2, 20) nach Anspruch 7, wobei die aufzufindende Komponente (12, 13) eine Aufnahmehülse (13) für Stäbe und/oder stabartige Teile, insbesondere für Füße von Eissporttoranlagen, umfasst.

9. Auffindevorrichtung (2, 20) nach Anspruch 8, wobei die Aufnahmehülse (13) das Magnetfeld (19) bereitstellt und/oder das Magnetfeld (19) durch mindestens einen Magneten (10, 11), insbesondere Ring- und/oder Hohlmagneten (11), welcher bei, an, in und/oder um die Aufnahmehülse (13) angeordnet ist, insbesondere in Bezug auf die Außenseite der Aufnahmehülse (13) um die Aufnahmehülse (13) herum und/oder koaxial zu dieser, insbesondere unter weniger als 10 Grad Abweichung, angeordnet ist, bereitgestellt wird.

10. Auffindevorrichtung (2, 20) nach einem der Ansprüche 7 - 9, umfassend mindestens einen magnetischen und/oder magnetsensitive/n Kugel und/oder Ball, insbesondere mehrere magnetische und/oder magnetsensitive Kugeln und/oder Bälle, insbesondere Kugel/n und/oder Ball/Bälle, welche mindestens einen Bewegungsfreiheitsgrad aufweisen, insbesondere wobei durch ein Rollen der Kugel/n und/oder des Balles/der Bälle auf einen Ort und/oder eine Ausrichtung des mindestens einen Magneten (10, 11) geschlossen werden kann.

11. Auffindevorrichtung (2, 20) nach einem der Ansprüche 7 - 10, wobei die Auffindevorrichtung (2, 20) ferner ein Prüfgerät zur horizontalen und/oder vertikalen Ausrichtung, insbesondere eine Wasserwaage, aufweist, insbesondere ferner eine zusätzliche Fläche für Kugeln und/oder Bälle aufweist, welche mit dem Prüfgerät im Schwerefeld ausgerichtet werden kann.

12. Auffindbare Vorrichtung (10 - 13) für den Winter- und Eissport zur verbesserten Lokalisierbarkeit von einer aufzufindenden Komponente (12, 13), insbesondere eissportrelevanten Befestigungskomponente (13), unter einer Eissportfläche (100), umfassend
mindestens eine aufzufindende Komponente (12, 13), insbesondere eine Aufnahmehülse (13) für Stäbe und/oder stabartige Teile, insbesondere für Füße von Eissporttoranlagen,
mindestens einen Magneten (10, 11), insbesondere einen Hohlmagneten (11), insbesondere einen Hohlmagneten, welche um die aufzufindende Komponente (12, 13), insbesondere die Aufnahmehülse (13) herum und/oder im Wesentlichen koaxial zu dieser, insbesondere unter weniger als 10 Grad Abweichung, angeordnet ist.

13. Auffindbare Vorrichtung (10 - 13) nach Anspruch 12 für den Winter- und Eissport zur verbesserten Lokalisierbarkeit von einer aufzufindenden Komponente (12, 13), welche mittels des vom mindestens einen Magneten (10, 11) bereitgestellten Magnetfelds (10, 11) eine Bestimmung einer Position, Ausrichtung und/oder räumlichen Orientierung der aufzufindenden Komponente (12, 13), insbesondere der Aufnahmehülse (13) für Stäbe und/oder stabartige Teile, ermöglicht.

14. Auffindbare Vorrichtung (10 - 13) nach Anspruch 12 oder 13, wobei die aufzufindende Komponente (12, 13) eine Aufnahmehülse (13) für Stäbe und/oder stabartige Teile, insbesondere für Füße von Eissporttoranlagen, umfasst.

15. Auffindbare Vorrichtung (10 - 13) nach Anspruch 14, wobei die Aufnahmehülse (13) das Magnetfeld (19) bereitstellt und/oder das Magnetfeld (19) durch mindestens einen Magneten (10, 11), insbesondere Ring- und/oder Hohlmagneten(11), welcher bei, an, in und/oder um die Aufnahmehülse (13) angeordnet ist, insbesondere in Bezug auf die Außenseite der Aufnahmehülse (13) um die Aufnahmehülse (13) herum und/oder im Wesentlichen koaxial zu dieser, insbesondere unter weniger als 10 Grad Abweichung, angeordnet ist, bereitgestellt wird.
